# EUROPÄISCHE PATENTSCHRIFT

(11) **EP 2 694 162 B1**
(45) Veröffentlichungstag und Bekanntmachung des Hinweises auf die Patenterteilung: **15.08.2018**
(21) Anmeldenummer: 11804680.4
(22) Anmeldetag: 21.12.2011
(51) Int. Cl.: A61Q 15/00, A61K 8/891, A61K 8/892, A61K 8/04

(54) **AEROSOLZUBEREITUNGEN MIT SILIKON GUMS UND SPEZIELLEN KOHLENWASSERSTOFF-GEMISCHEN**
AEROSOL FORMULATIONS COMPRISING SILICONE GUMS AND SPECIAL HYDROCARBON MIXTURES
PRÉPARATIONS EN AÉROSOL COMPORTANT DES GOMMES SILICONES ET DES MÉLANGES HYDROCARBURES SPÉCIFIQUES

(30) Priorität: 21.12.2010 DE 102010063754
(43) Veröffentlichungstag der Anmeldung: 12.02.2014
(73) Patentinhaber: Beiersdorf AG, 20253 Hamburg (DE)
(72) Erfinder: MIERTSCH, Heike, 22459 Hamburg (DE); RIPKE, Sabine, 22527 Hamburg (DE); BIEL, Stefan, 21077 Hamburg (DE); SCHOLZ, Annika, 22459 Hamburg (DE); URBAN, Michael, 22523 Hamburg (DE)
(74) Vertreter: Hartmann, Jost
(86) Internationale Anmeldenummer: PCT/EP2011/073574
(87) Internationale Veröffentlichungsnummer: WO 2012/085074

(56) Entgegenhaltungen:
- EP-A1- 2 189 149
- EP-A2- 0 343 843
- EP-A2- 0 452 762
- WO-A1-98/35648
- WO-A1-2005/063188
- WO-A1-2010/054921
- GB-A- 1 555 044
- US-A- 4 152 416
- US-A- 5 082 652
- US-A- 5 800 805
- US-A- 6 093 408
- US-A- 6 096 325
- US-A1- 2006 104 918
- L. Durand ET AL: "Influence of different parameters on droplet size and size distribution of sprayable sunscreen emulsions with high concentration of UV-filters", INTERNATIONAL JOURNAL OF COSMETIC SCIENCE., vol. 29, no. 6, 8 March 2007 (2007-03-08), pages 461-471, XP55390246, NL ISSN: 0142-5463, DOI: 10.1111/j.1468-2494.2007.00408.x
- Anonymous: "DOW CORNING 1501 Fluid Personal Care Product Information TYPICAL PROPERTIES", , 20 April 1998 (1998-04-20), XP55390610, Retrieved from the Internet: URL:http://www.corquiven.com.ve/esp/PDS/SI LICON_1501_Fluid.pdf [retrieved on 2017-07-13]

## Beschreibung

Die Erfindung umfasst Aerosolzubereitungen mit Silikon Gums und einem oder mehreren Kohlenwasserstoff-Gemischen mit einem Anteil an Kohlenwasserstoffen mit einer Kettenlänge größer und/oder gleich C16 von mindestens 50 Gew.%, bezogen auf die Gesamtmasse des Kohlenwasserstoffgemisches.
Die Kombination führt neben verbesserten Sprüheigenschaften auch zu verbesserten sensorischen Eigenschaften der Aerosolzubereitungen.
Ein Aerosol ist ein disperses System, bei dem ein Feststoff oder eine Flüssigkeit in einem Gas äußerst fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Gemisch aus Treibmittel und Wirkstofflösung durch eine feine Düse, das Treibmittel verdampft und hinterlässt das fein verteilte Sprühgut als Aerosol. Im anwendungstechnischen Sprachgebrauch wird der Begriff Aerosol vom Fachmann häufig auch im Sinne von Aerosolsprays verwendet, d.h. hier wird unter "Aerosol" nicht der reine Sprühnebel, sondern eine Druckgasverpackung nebst Abgabe- bzw. Dosiervorrichtung und Füllgut verstanden.
Das Füllgut umfasst Wirkstofflösung und Treibmittel. Als Wirkstofflösung wird die Summe aller Zubereitungsbestandteile ohne das Treibmittel bezeichnet. Der Begriff Wirkstofflösung wird auch dann verwendet, wenn die Zubereitung z.B. in Form einer Suspension oder Emulsion vorliegt.
Als Suspension ist eine Dispersion zu verstehen, bei der die kontinuierliche Phase im flüssigen und die dispergierte Phase im festen Zustand vorliegen.
Als Sprühgut, Sprühnebel, Sprühstoß oder Sprühkegel wird synonym das bei einmaliger Betätigung der Aerosolvorrichtung ausgetriebene Füllgut betrachtet.
Üblicherweise werden Antitranspirantien (AT) und Desodorantien (Deo) in mannigfaltigen Produktformen angeboten, wobei in Europa Roller, Pumpzerstäuber und Aerosole dominieren, in den USA, Mittel- und Südamerika eher die Stifte. Es sind sowohl wasserfreie (Suspensionen) als auch wasserhaltige Produkte (hydro-alkoholische Formulierungen, Emulsionen) bekannt.

Antitranspirantien oder Desodorantien dienen dazu, Körpergeruch zu beseitigen oder verhindern, der entsteht, wenn der an sich geruchlose frische Schweiß durch Mikroorganismen zersetzt wird. Den üblichen kosmetischen Desodorantien liegen unterschiedliche Wirkprinzipien zugrunde.
Im allgemeinen Sprachgebrauch erfolgt nicht immer eine klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.
Im Stand der Technik finden sich bei wasserfreien Suspensionen fast ausschließlich Deodorant- oder Antitranspirantformulierungen, die Silikonöle, insbesondere Cyclomethicone, beinhalten.
Flüchtige Silikonöle, insbesondere Cyclomethicone, dienen als Trägeröl für die Wirkstoffe und ermöglichen so ein gleichmäßiges Aufbringen auf die Haut. Durch die hohe Flüchtigkeit dampft Cyclomethicone sehr schnell von der Haut ab, ohne ein klebriges Gefühl zu verursachen. Durch das Abdampfen des Silikonöls werden weiße Rückstände durch das AT-Mittel oder die Verdicker auf der Haut sehr schnell sichtbar (Weißeleffekt). Formeln mit Cyclomethiconen werden durch viele Verbraucher als zu trocken und wenig pflegend empfunden.
Cyclomethicone, z.B. INCI-Bezeichnung für ein Octamethylcyclotetrasiloxan, besitzen die allgemeine Formel, mit I = 3 - 6 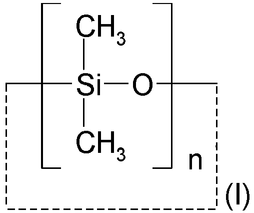 Angeboten werden beispielsweise Cyclomethicon/Decacyclopentasiloxan (D5) oder Cyclomethicon/Dodecamethylcyclohexasiloxan (D6). Die Gemische können für sich allein oder in entsprechenden Zubereitungen als flüchtige Silikonverbindungen eingesetzt werden (Cosmet. Toiletries 107, Nr. 5, 27 [1992]).
Nachteile der Cyclopentasiloxane (D5) und der flüchtigen Silikonöle allgemein sind:
a) Verfügbarkeit
   Durch hohen Verbrauch von Silikonölen in der Photovoltaik und in der Baustoffindustrie ist eine permanente Versorgung mit Silikonölen für die Kosmetik nicht ungehindert gewährleistet.
b) Nachhaltigkeit
   Silikonöle zeichnen sich häufig durch eine mangelnde Abbaubarkeit aus.
c) Weißeleffekt
   Silikonölhaltige Formulierungen zeigen den Nachteil des Weißelns von Antitranspirantien auf insbesondere schwarzer Baumwollkleidung. Silikonölfreie Formulierungen mit gleicher Konzentration des Antitranspirantwirkstoffs in Form eines Pulvers zeigen ein deutlich geringeres Weißeln.
d) Pflegegefühl
   Silikonöle in Kosmetika vermitteln dem Verbraucher ein geringes Pflegegefühl.

Silikonöle haben auch deutliche Schwächen in Bezug auf Ihre Kompatibilität mit kosmetischen Wirkstoffen. Aufgrund ihres stark hydrophoben Charakters vermögen Silikonöle insbesondere Wirkstoffe mit polarem Charakter kaum zu lösen. Weiterhin sind Silikonöle praktisch nicht in der Lage, die Penetration kosmetischer Wirkstoffe in die Haut zu unterstützen.
Zu den silikonhaltigen Ölen zählen auch die als Silikon Gums bezeichneten hochmolekularen Polydiorganosiloxane. Silikon Gums sind beispielsweise in der US 4152416 beschrieben. Hochmolekular bedeutet eine Molmasse von ca. 100.000 bis 2.000.000 g/mol.
Bekannte Silikon Gums sind polymere Polydimethylsiloxane (INCI-Bezeichnung: Dimethicone), z. B. die unter der Bezeichnung DC 1411 Fluid und DC 1413 Fluid von Dow Corning vertriebenen Produkte, und Dimethylsiloxane mit Hydroxy-Endgruppen (INCI-Bezeichnung: Dimethiconol), z. B. die Handelsprodukte DC 1501.
Für kosmetische Anwendungen weitere bekannte Silikon Gums sind beispielsweise SF1236, SF1276, CF1251 von Momentive Performance Materials.

Bekannt sind des Weiteren die Silikon Gums der Fa. Dow Corning mit den Bezeichnungen Dow Corning® 1501 Fluid (Cyclopentasiloxane, Dimethiconol), Dow Corning® 1503 Fluid (Dimethicone, Dimethiconol), Dow Corning® CB 1556 Fluid (Phenyl Trimethicone, Dimethiconol) und Dow Corning® BY 25-320 (C13-16 Isoparaffin, Dimethicone). Mischungen von Silikonflüssigkeiten und flüchtigen Silikonölen, wie Cyclomethicone, und Silikon Gums sind ebenfalls bekannt in kosmetischen Zubereitungen.
Das im Handelsprodukt DC 1503 zu 88% enthaltene Dimethicone hat ein Molekulargewicht unter 100.000 g/mol und ist somit kein erfindungsgemäßes Silikon Gum.

WO 9804236 A1 beschreibt Antitranspirantien (AT) mit Silikonlatex und weiteren Verdickern, wie u.a. Silikon Gum.

EP 452762 A2 beschreibt AT-Aerosole umfassend Silikongummi und Silica, Lösemittel, AT-Salz und ein Treibmittel, wobei eine Kombination von Silica und Silikongummi als Suspendierhilfsmittel eingesetzt wird.

WO 2005074877 A1 beschreibt Aerosol-Antitranspirantien mit gegenüber den AT-Mitteln inerten Silikonen.

WO 2005063188 A1 beschreibt silikonfreien AT-Sprays.

DE 102005047370 A1 offenbart AT-haltige W/O-Emulsionen (Sprays), bei denen das AT-Mittel gelöst in der inneren Phase vorliegt.

DE 69823490 T2 beschreibt wasserfreie AT-Aerosole mit 5-25% Pentan und Silikongummi, welches zur Verstärkung der kühlenden Eigenschaften eingesetzt wird.

US 4152416 A lehrt AT-Aerosole mit geringer Nebel- und Staubbildung mit AT-Salz, Treibgas, Suspendierhilfe und einem synthetischen Polymergummi (500.000 bis 100 Mio. cSt).

EP 343843 B1 beschreibt AT-Aerosole mit einer Sprührate von nicht mehr als 0,5 g/s mit AT-Salz, Treibgas und einer Kombination aus Silikonpolymer und einer flüchtigen niedrigviskosen Flüssigkeit.

WO 2010/054921 A1 offenbart eine Aerosolformulierung mit einem Silikongummi.

Es ist bekannt, dass sich die Tröpfchengröße eines Aerosolsprühstosses zu einem gewissen Maße mit dem Abfüllverhältnis von Wirkstofflösung zu Treibgas steuern lassen kann. Erhöht man den Anteil der Wirkstofflösung, so vergrößert sich der Durchmesser der ausgetragenen Tröpfchen.
Nachteilig dabei ist aber eine Verteuerung des Produktes. Zudem hat ein höherer Anteil an Wirkstofflösung im Abfüllverhältnis einen negativen Einfluss auf das Hautgefühl, da dadurch ein nasses und kaltes Gefühl auf der Haut erzeugt werden kann, welches in vielen Anwendungsbereichen nicht erwünscht ist. So ist z.B. ein Vorteil von Antitranspirantien und Deodorantien in Aerosolform gerade das trockene Hautgefühl.

Zudem kann das Produkt durch den Auftrag von mehr Wirkstofflösung auf der Haut große Tropfen bilden, die dann auf der Haut nach unten rinnen, so dass eine gleichmäßige Verteilung des Produktes auf der Haut nicht mehr gewährleistet ist.
Auch durch das Ventil der Aerosolsprühapparatur kann die Tröpfchengröße beeinflusst werden. Verringert man den Durchmesser der Gehäuseseitenbohrung wird weniger Gasphase beim Austragen des Produktes zugemischt, was wiederum eine Erhöhung des Anteils an Wirkstofflösung in dem ausgetragenen Gas/Wirkstoffgemisch verursacht. Dies führt zwar zu einer Vergrößerung des Tröpfchendurchmessers, zeigt aber auch die oben beschriebenen Nachteile wie bei einer Erhöhung des Wirkstoffanteils beim Abfüllverhältnis.
Des Weiteren ist es möglich durch Variation des Querschnitts der Kegelbohrung im Ventil die Partikelgrößenverteilung zu beeinflussen, was jedoch an eine Veränderung der Sprührate gekoppelt ist.
Die Sprührate ergibt sich aus dem Mittelwert der Austragsmenge der ersten 3 Sprühstöße zu je 10 Sekunden von mindestens einer vollen (unbenutzten, nicht angesprühten) Dose. Die Austragsmenge beschreibt die Menge an abgegebenem Gas/Wirkstoffgemisch und wird durch Rückwiegen der Dose ermittelt. Die Bestimmung erfolgt bei Raumtemperatur.
Um eine neue Aerosolzubereitung in den Verkehr zu bringen sind eine sorgfältige Auswahl von Inhaltsstoffen und eine umfangreiche Testung des Produktes auch unter Sicherheitsaspekten erforderlich. Zur Beurteilung der Sicherheit und Anwendungsfreundlichkeit eines Aerosolsprays gehört u.a. die Untersuchung der Partikelgrößen im Sprühnebel. Dies ergibt sich u.a. aus Anhang 2, EG-Aerosolrichtlinie, 2008/47/EG vom 8. April 2008 (ABI. Nr. L 96/15).
Hierbei ist insbesondere ein möglichst geringer Anteil der Partikel unter 10µm Partikelgröße wünschenswert. Die Relevanz dieser Größenklasse ergibt sich aus der allgemeinen Diskussion um Feinstäube, bei der Partikel unter 10µm als PM10-Feinstaub bezeichnet werden.
Wünschenswert ist es daher, eine Möglichkeit zu finden, mit der der Feinanteil im Aerosolsprühstoß reduziert werden kann, ohne negative Auswirkungen auf Hautgefühl und Formelkosten zu verursachen.
Die Erfindung umfasst kosmetische oder dermatologische wasserfreie Aerosolzubereitungen gemäß Anspruch 1. Die Zubereitungen führen zur Verbesserung des Sprühkegels und der Verringerung von Satellitenpartikeln, was ein gezieltes Auftragen des Aerosolsprays ermöglicht.
Unter Satellitenpartikeln sind Partikel zu verstehen, die sich außerhalb des direkten Applikationsstrahles, beispielsweise durch Verwirbelungen, bilden.
Als Partikel sind die flüssigen oder festen Teile des Aerosolsprühstoßes zu verstehen.

Bei sachgemäßer Anwendung von Aerosolpackungen ergibt sich ein Sprühstoß, Sprühkegel oder Sprühstrahl. In diesem wird erfindungsgemäß der Feinanteil von Partikeln durch den Zusatz an Silikon Gums verringert.
Der Feinanteil von Partikeln kann beispielsweise gebildet sein aus festen Inhaltsstoffen der Zubereitungen, wie z.B. Antitranspirant-Wirkstoffe, wie ACH, oder Suspendierhilfen, aber auch aus flüssigen Bestandteilen wie Träger- und Maskierungsöle sowie verflüssigtem Treibgas sowie aus Mischungen dieser Bestandteile.
Bevorzugt liegt die Sprührate eines Aerosol-Produktes mit der erfindungsgemäßen Aerosolzubereitung im Bereich von 5 bis 12 g/10s, bevorzugt 6,5 bis 10 g/10s.
Überraschend zeigte sich auch im Hinblick auf die zuvor geschilderten Nachteile von Silikonölen, dass bei der Verwendung von Silikon Gums in Kombination mit den Kohlenwasserstoff-Gemischen die erfindungsgemäßen Zubereitungen dennoch eine gute Wirkstoffverfügbarkeit und angenehme sensorische Eigenschaften aufweisen.
Silikon Gums können als Demisting Agents in Sprays eingesetzt werden, um die Vernebelung zu verringern und den Sprühkegel enger zu machen. So kann das Aerosol Spray fokussierter auf die Achsel aufgebracht werden.
Dass Silikon Gums zusätzlich in der Lage sind, den Anteil an Partikeln mit einer Partikelgröße kleiner 10µm deutlich zu verringern, ist dagegen überraschend und erfindungsgemäß von Vorteil.
Im Stand der Technik zu Aerosolen, wie in US 4152416 A, wird beschrieben, dass die Erhöhung der Viskosität des Sprühgutes zu einer Verringerung der Nebel- und Staubbildung führt. Untersuchungen haben jedoch gezeigt, dass die Viskosität der Wirkstofflösung dabei nicht im direkten Zusammenhang mit dem Anteil an kleinen Partikel (< 10µm) im Sprühstoß steht. Es zeigt sich, dass die Erhöhung der Viskosität der Wirkstofflösung keine Auswirkungen auf die Partikelgröße im Sprühstoß zeigt.

In weiteren Untersuchungen zeigte sich, dass 0,75 Gew.% Aktivgehalt eines Silikon Gums, bezogen auf die Gesamtmasse der Wirkstofflösung, insbesondere DC 1501-Fluid, dass dann zu 5 Gew.% enthalten ist (entsprechend einem Aktivgehalt an Silikon Gum von 0,75% in der Wirkstofflösung ohne Treibgas), in der Lage ist, bei einem marktüblichen Antitranspirant Spray den Anteil an Partikeln mit einer Partikelgröße kleiner 10µm um etwa die Hälfte zu reduzieren.
Bei einem Treibgasanteil von ca. 85% ergibt sich dann der erfindungsgemäß vorteilhafte Anteilsbereich von ca. 0,1125 Gew.% Silikon Gum, bezogen auf die Gesamtmasse der Zubereitung.

Die untersuchten Rezepturen entsprechen den Beispielrezepturen Standard (Stand der Technik) und Beispielen 1, 2, 3, 24, 25 und 26.
Der Anteil an Aktivgehalt Silikon Gum liegt erfindungsgemäß im Bereich von 0,02 - 0,5 Gew.%, bezogen auf die Gesamtmasse der Zubereitung, besonders bevorzugt im Bereich von 0,035 % bis 0,2 Gew.%, insbesondere im Bereich von 0,04 bis 0,1 Gew.%, bezogen auf die Gesamtmasse der Zubereitung incl. Treibmittel.
Überraschenderweise wird das Hautgefühl durch den Zusatz an Silikon Gum nicht negativ beeinflusst.
Als Silikon Gums gelten erfindungsgemäß hochmolekulare Polydiorganosiloxane, Dimethicone und Dimethiconol. Hochmolekular bedeutet eine Molmasse von ca. 100.000 bis 2.000.000 g/mol.
Bevorzugt werden als erfindungsgemäße Silikon Gums gewählt:
- DC 1501 Fluid (INCI: Cyclopentasiloxane (and) Dimethiconol) 15% Dimethiconol (Aktivgehalt), 85% Cyclopentasiloxane
- DC 1411 Fluid (INCI: Cyclopentasiloxane (and) Dimethicone) 15% Dimethicone (Aktivgehalt), 85% Cyclopentasiloxane.
- DC 1503 Fluid (INCI: Dimethicone (and) Dimethiconol) 12% Dimethiconol (Aktivgehalt), 88% Dimethicone
- DC BY 25-320 (INCI: C13-16 Isoparaffin (and) Dimethicone (and) C10-13 Isoparaffin) 20% Dimethicone Gum (Aktivgehalt), 80% Isoparaffin
- DC 1413 Fluid (INCI: Dimethicone) 14% Aktivgehalt Dimethicone Gum

Zudem werden die Silikon Gums in der Kosmetik hauptsächlich auf Grund ihrer Eigenschaft eingesetzt, ein weiches, seidiges Hautgefühl zu verleihen, was auch die erfindungsgemäßen Zubereitungen weiter verbessert.

Die Messung der Partikelgröße der in den Abbildungen dargestellten Untersuchungen erfolgte mittels Bestimmung der Laserbeugung mit dem Particlesizer Insitec Measurement System der Firma Malvern mit den Parametern:
- Brennweite Linse 200 mm
- Abstand Aerosoldüse zu Linse: 15 cm
- Abstand Aerosoldüse zum Laserstrahl: 15 cm
- Dosenhöhe: der Laser soll mittig durch den Sprühstrahl gehen
- Dauer der Messung: 2 Sekunden
- Temperatur: < 22°C
- Auswertung anhand von der Software Insitec RT Sizer für Windows Version 5.6 Einstellungen:
- Weglänge: 12,00 mm
- Sattering Rings: 1 - 31
- Size Bins: 3 - 50
- Dichte des Partikels 1,00 (gm/cc)
- Threshold = 20,0
- Multiple Scattering: on
- Mesh factor = 1
- Flash Settings:
- Flash Aufnahme : 1 Scans : 200 Hz : DC = 0,0%
- Dauer: 5,0 ms

Der in den Abbildungen angegebene Messwert ist die Volumen Häufigkeit der Partikel in %, die eine Größe kleiner 10µm haben. Es wird vorzugsweise ein Durchschnittswert von drei vermessenen Aerosoldosen gebildet, die jeweils drei Mal vermessen wurden und ergibt sich demnach aus insgesamt neun Messungen. Der durch das verdampfende Treibgas und somit den sich ständig verändernden Brechungsindex erzeugte zusätzliche Gaspeak (Beam Steering) wird bei der Auswertung der Daten nicht berücksichtigt.
Die Partikelgröße ist nach DIN 66160: 1992-09 Absatz 2.2.5 "ein Feinheitsmerkmal der Dimension Länge". Gemeint ist damit zunächst der Partikeldurchmesser, was strenggenommen nur für kugelförmige Partikel gilt bzw. bei isometrischen Partikeln zumindest sinngemäß angewandt werden kann. Im Falle nicht-isometrischer Partikel gilt als Partikelgröße ein Mittelwert der Teilchenabmessungen (Römpp Online 3.10).

Insbesondere Aerosolzubereitungen mit einem partikulären Wirkstoffanteil, wie beispielsweise wasserfreie Antitranspirantzubereitungen, lassen sich mit Hilfe des Silikon Gums in ihren Sprüheigenschaften verbessern. D.h. bei Wirkstofflösungen in Form einer Suspension ist die Verwendung von Silikon Gums zur Verringerung des Anteils an Partikeln mit einer Partikelgröße kleiner 10 µm im Aerosolsprühstoß besonders vorteilhaft.

D.h. der Sprühstoff kann fokussierter gestaltet werden, was zu einer Applikation der Wirkstoffe an den Orten führt, an dem die Wirkstoffe auch aufgebracht werden sollen. Eine Vernebelung der Umgebung wird vermieden. Darüber hinaus wird der Feinanteil an Partikeln im Sprühstoß der Aerosol Sprays verringert, d.h. die Anzahl an Partikeln mit einer Partikelgröße kleiner 10 µm wird vermindert.

Zum Unterschied der, als Partikel bezeichneten, flüssigen Sprühnebelbestandteile sind bei den Wirkstoffen, wie AT-Wirkstoffen, deren festen Teile (in Suspension) als Partikel zu verstehen. Weitere partikuläre Bestandteile der Zubereitung können Suspendierhilfen oder andere puderförmige Zusatzstoffe sein.

Suspendierhilfen erhöhen die Stabilität von Partikeln in Suspensionen. In wasserfreien Antitranspirantformulierungen werden vorzugsweise modifizierte Schichtsilikate, Tonmineralien und/oder Kieselsäuren als Suspendierhilfen eingesetzt. Modifizierte Schichtsilikate im Sinne der vorliegenden Erfindung sind Schichtsilikate, insbesondere die bereits genannten Schichtsilikattypen, deren Organophilie (auch: Lipophilie) - z. B. durch Umsetzung mit quarternären Ammonium-Verbindungen - erhöht wurde. Solche Schichtsilikate werden auch als organophile Schichtsilikate bezeichnet. Besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18. Bei der Verwendung von Tonmineralien kann zusätzlich ein sogenannter Aktivator verwendet werden.
Bevorzugt sind die Suspendierhilfsmittel, insbesondere Schichtsilikate, insbesondere Disteardimonium Hectorite, zu einem Anteil von bis zu 1 Gew.%, insbesondere 0,3 bis 0,6 Gew.%, bezogen auf die Gesamtmasse der Zubereitung enthalten.

Wasserfrei bedeutet, dass der Anteil an Wasser in den Aerosolzubereitungen, abgesehen von ggf. mit eingeschleppten Verunreinigungen oder Kristallwasser, nahezu 0 Gew.% beträgt.
Erfindungsgemäß sind Aerosolzubereitungen umfassend ein oder mehrere Silikon Gums und ein oder mehrere Kohlenwasserstoffgemische mit einer überwiegenden Kettenlänge ≥ C16.
Überwiegend bedeutet erfindungsgemäß, dass der Anteil an Bestandteilen im Kohlenwasserstoffgemisch mit Kettenlängen größer/gleich C16 mindestens 50%, bevorzugt mindestens 60%, beträgt. Kohlenwasserstoffgemische, die ausschließlich aus Bestandteilen mit C16 als Kettenlängen bestehen, sind demnach nicht erfindungsgemäß.
Ebenso bevorzugt ist ein Kohlenwasserstoff-Gemisch mit einer Kohlenstoffanzahl Cx mit x =16 + 4*a, wobei a aus der Menge der natürlichen Zahlen N = {0, 1, 2, 3,....} gewählt wird. Der Anteil an Kohlenwasserstoffen mit a = 0 beträgt mindestens 50 Gew.%, bevorzugt mindestens 55 Gew.%, insbesondere mindestens 60 Gew.%, bezogen auf die Gesamtmasse des Kohlenwasserstoffgemisches.
Das Kohlenwasserstoffgemisch Cx ist aus Oligomeren des Buten bzw. Butadien gebildet. Erfindungsgemäß bevorzugt beträgt der Anteil an Kohlenwasserstoffen Cx mit a ≥ 1 mindestens 0,5 Gew.% und mit a > 4 (d.h. KW >C28) weniger als 0,5 Gew.%, jeweils bezogen auf die Gesamtmasse des Kohlenwasserstoffgemisches. D.h. es sind mindestens 0,5 Gew.% an C20, C24, C28 oder C30 Kohlenwasserstoffen enthalten. Der Anteil an C32, C36 etc. beträgt weniger als 0,5 Gew.%.
Der Anteil an Bestandteilen im Kohlenwasserstoffgemisch Cx mit Kettenlängen gleich C16 beträgt mindestens 50%, bevorzugt mindestens 60%. Kohlenwasserstoffgemische, die ausschließlich aus Bestandteilen mit C16 als Kettenlängen bestehen, sind dabei nicht erfindungsgemäß bevorzugt.
Der Fachmann erwartete allerdings eine begrenzte Löslichkeit von Silikon Gums in den bevorzugten nichtflüchtigen Ölen Isopropylpalmitat, Alkylbenzoat, Caprylic/Capric Triglycerid und/oder lineare/verzweigten Kohlenwasserstoffölen mit einer überwiegenden Kettenlänge ≥ C16. Jedoch ist es überraschender Weise gelungen, die Silikon Gums stabil und wirksam einzuarbeiten. Erfindungsgemäß zeigen sich Kohlenwasserstoffe z. B. C14-22 Alkane mit überwiegender Kettenlänge ≥ C16 oder die bevorzugten KW-Gemische Cx darüber hinaus als kostengünstige Alternative.
Lipide bezeichnen Fette, fettähnliche Stoffe und Öle. Für die Kosmetik sind sie vor allem als weichmachende ("emollient") Inhaltsstoffe und als hauteigene Lipide der Hornschicht, die zwischen den Hornzellen lagern, von Bedeutung. Sie befähigen die Haut zur Speicherung von Feuchtigkeit. Neben dem pflegenden Aspekt werden Lipide den kosmetischen Zubereitungen zugesetzt, um eine bessere Verteilbarkeit auf der Haut zu gewährleisten und um die sensorischen Eigenschaften der Zubereitungen zu verbessern.
Durch den Einsatz dieser Lipide wird zum einen ein zusätzlich pflegendes Hautgefühl vermittelt und zudem die weißen Rückstände des Aluminiumchlorohydrates maskiert. Das bedeutet, dass weniger weiße Rückstände auf der Haut und schwarzer Kleidung sichtbar sind.
Wie zuvor dargestellt, werden im Stand der Technik leicht flüchtige Silikonöle, wie Cyclomethicone als Hauptbestandteil in Aerosolsprays eingesetzt. Durch die Verwendung der genannten erfindungsgemäßen Kohlenwasserstoff-Gemische kann der Anteil an Cyclomethicone deutlich reduziert werden bzw. Cyclomethicone kann komplett ersetzt werden.
AT-Sprays, bei denen Cyclomethicone durch eines oder eine Mischung der erfindungsgemäßen Kohlenwasserstoff-Gemische teilweise oder vollständig ausgetauscht worden ist, wurden durch Verbraucher ebenso gut wie das Vergleichsprodukt mit Cyclomethicone beurteilt, wie die folgenden Untersuchungen zeigen.

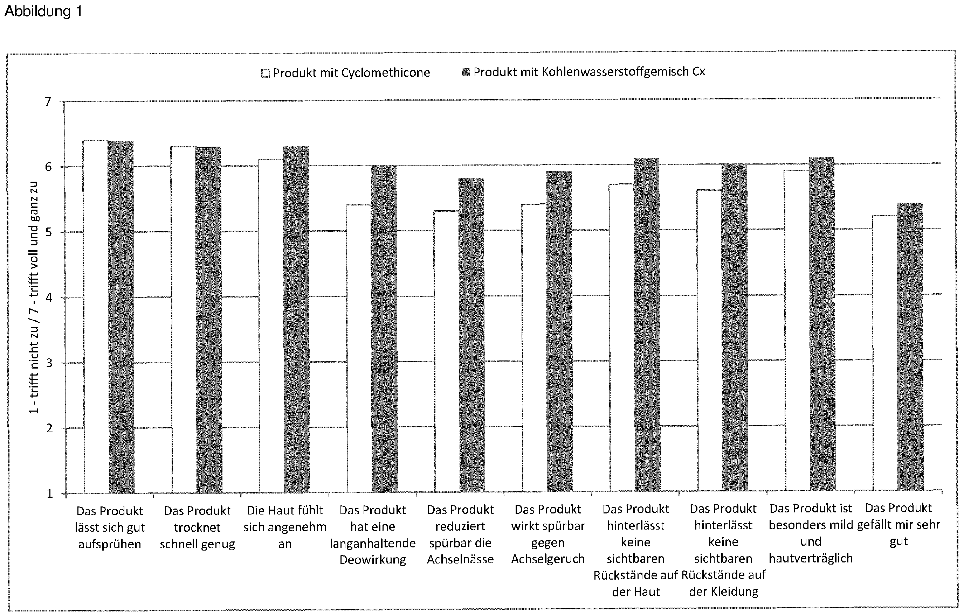

Abbildung 1 zeigt das Ergebnis eines In-Use-Tests mit 53 Probandinnen, die semimonadisch erst eine Woche die Neuentwicklung des wasserfreien Antitranspirantsprays mit einem Kohlenwasserstofflipid und Triglycerid und anschließend eine Woche als Referenz das Vergleichsprodukt mit dem Silikonöl Cyclomethicone getestet haben. Abschließend wurden die genannten Punkte in einem Fragebogen von den Probandinnen beantwortet (weiße Balken: Vergleichsprodukt mit Cyclomethicone; dunkle Balken: Neuentwicklung mit Silikon Gum, Kohlenwasserstofflipid und Triglycerid).
Kohlenwasserstoffe haben allgemein den Nachteil, dass sie eine sehr schlechte Packmittelkompatibilität haben. Schon nach kurzer Lagerzeit migrieren die Kohlenwasserstoffe in das Packmittel und können so die Funktion des Packmittels einschränken. Bei den Aerosolen ist eine Inkompatibilität der Öle mit den Kunststoffteilen des Ventils und des Sprühkopfes denkbar.

Die erfindungsgemäß bevorzugten Kohlenwasserstoffe mit einem Anteil von mindestens 50% an Kohlenwasserstoffen mit einer Kettenlänge größer/gleich C16, wie z.B. C14-22 Alkane oder die KW-Gemische Cx zeigen nun die Vorteile von der Rohstoffgruppe der Kohlenwasserstoffe, wie z.B. Stabilität in Marktformeln nach Austausch von Cyclomethiconen, eine vergleichbare Sensorik wie mit Cyclomethiconen, während sie den typischen Nachteil der Packmittelinkompatibilität nicht oder erst nach Lagerung über einen sehr langen Zeitraum (z.B. 1 Jahr) bei einer Temperatur, die deutlich über +25°C (z.B. +35°C, +40°C, +45°C) liegt. Der Hauptunterschied liegt hier in der Kinetik, mit der der Kohlenwasserstoff in das Packmittel migriert. Die Kinetik ist deutlich verlangsamt, so dass der Verbraucher das Produkt über Jahre bei +25°C lagern kann, ohne dass es zu den für Kohlenwasserstoffen typischen Packmittelverformungen kommt.
Um die Packmittelkompatibilität der erfindungsgemäßen Zubereitungen zu ergründen, wurden Tests durchgeführt.
Dazu wird das Ventil/der Sprühkopf einer Aerosoldose in einem Glasgefäß in das entsprechende Öl getaucht. Das Glas wird mit einem Schraubdeckel verschlossen und für 6 Tage bei +45°C gelagert.
Anschließend werden die Ventile/die Sprühköpfe auf Veränderungen untersucht. Die so behandelten Ventile/Sprühköpfe werden anschließend auf ihre Funktionsfähigkeit getestet. Es zeigt sich, dass die Packmittelkompatibilität der erfindungsgemäßen Formulierungen, insbesondere mit C14-22 Alkanen, sehr gut ist, die Kinetik der Migration des Öls in das Packmittel deutlich langsamer ist als bei anderen Kohlenwasserstoffen wie Isohexadecane oder Isododecane und der Polypropylen-Sprühkopf sitzt weiterhin fest auf der Dose.
Besonders positiv hat sich eine Mischung aus Kohlenwasserstoffen mit einem Anteil von mindestens 50% an Kettenlängen größer/gleich C16 gezeigt. Während Kohlenwasserstoffmischungen mit überwiegend kürzerkettigen Anteilen (z.B. C13-16 Isoparaffin) und Kohlenwasserstoffmischungen, die nahezu keine Anteile an Kettenlängen größer C16 enthalten, wie zum Beispiel Isododecane und Isohexadecane eine schlechtere Packmittelkompatibilität zeigen, hat diese spezielle Mischung eine außergewöhnlich gute Packmittelkompatibilität.
Auch zeigen die erfindungsgemäßen Kohlenwasserstoffe eine sehr gute Verträglichkeit, die in mehreren In-Use-Tests mit jeweils 60 Probandinnen und Kurzzeit-Applikation der Produkte von einer Woche als auch in in vitro-Tests (HET-CAM und RBC) bestätigt werden konnte.

Im Rahmen eines Cyclomethicone-Austauschs stellen die erfindungsgemäßen Kohlenwasserstoff-Gemische eine besonders kostengünstige Alternative dar. Da diese Rohstoffmischungen frei von Silikonen sind, stehen sie auch bei einer Knappheit von Silikonölen weiterhin als Basis für Antitranspirant-Formulierungen zur Verfügung.
Aufgrund des gezeigten Vorteils beim Austausch von Cyclomethiconen durch die erfindungsgemäßen Kohlenwasserstoff-Gemische, ist der Gehalt an Cyclomethiconen in der Zubereitung kleiner 8 Gew.%, bevorzugt kleiner 5%, besonders bevorzugt 0 Gew.%, bezogen auf die Gesamtmasse der Zubereitung inklusive Treibgas, zu wählen. Als Cyclomethicone sind insbesondere Cyclopentasiloxane (D5), Cyclohexasiloxane (D6) oder Mischungen daraus zu verstehen.
Für den Fachmann unerwartet, zeigte sich bei den erfindungsgemäßen Aerosolzubereitungen zudem ein besseres Absetzverhalten des partikulären Wirkstoffs im Aerosol bei gleichzeitig sehr guter Packmittelkompatibilität. Die Partikel sedimentieren deutlich langsamer, wenn Cyclomethicone (D5) gegen das Kohlenwasserstoffgemisch ausgetauscht worden ist.

Der Anteil des Kohlenwasserstoff-Gemischs in der Aerosolzubereitung beträgt vorteilhaft 0,3 bis 15,0 Gew.%, insbesondere 0,4 bis 10,0 Gew.%, bezogen auf die Gesamtmasse der Zubereitung.
Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen. Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.
Neuartige AT-Wirker basieren bspw. auf dem Prinzip der Anticholinergika, welche die Nervenreize, die zur Sekretionssteigerung der Schweißdrüsen führen, unterbrechen.
Ein weiteres Prinzip neuartiger AT-Wirker beruht auf der Beeinflussung von Membrantransportvorgängen in der Zelle. So hemmen spezifische Aquaporin-Inhibitoren die Proteine, die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmosevorgängen. Ein weiterer neuartiger AT-Wirkmechanismus lässt sich durch die Verwendung kurzkettiger, vicinaler Diole realisieren, welcher möglicherweise auf deren osmotischen Aktivität zurückzuführen ist.
So durch Anwendung eines Mittels kein Einfluss auf die Schweißsekretion ausgeübt wird, also keine antitranspirante Wirkung realisiert wird, liegt erfindungsgemäß kein Antitranspirant vor.
Erfindungsgemäß sind demnach als Antitranspirantwirkstoffe solche Stoffe umfasst, die einen Einfluss auf die Schweißsekretion haben.
Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Gängige Wirkmechanismen hierzu sind antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.
Zubereitungen können neben den eigentlichen schweißhemmenden Wirkstoffen (AT-Wirker) zusätzlich auch Stoffe enthalten, die den mikrobiellen Abbau des Schweißes hemmen, wie z. B. Butyloctanoic Acid. Butyloctanoic Acid zeigt jedoch nur eine desodorierende Wirkung und keine antitranspirante Wirkung, da aus der Beeinflussung der bakteriellen Hautflora keine Beeinflussung der Schweißsekretion abzuleiten ist.
Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.
Antitranspirantwirkstoffe im Sinne der vorliegenden Anmeldung sind insbesondere aus den folgenden Gruppen zu wählen.

Als Antitranspirantwirkstoffe finden insbesondere Adstringenzien Verwendung, vornehmlich Aluminiumverbindungen. Die früher eingesetzten, stark sauer wirkenden Salze Aluminiumsulfat oder -chlorid und die Agaricinsäure sind weitgehend durch Aluminiumhydroxychlorid und -alkoholate ersetzt worden. Die nachfolgende Auflistung vorteilhaft einzusetzender Antitranspirant-Wirker soll in keinster Weise einschränkend sein: Aluminiumsalze:
- Aluminiumsalze wie Aluminiumchlorid AlCl3, Aluminiumsulfat Al2(SO4)3
- Aluminiumchloride der empirischen Summenformel [Al2(OH)mCln], wobei m+n=6
- Aluminiumchlorhydrat [Al2(OH)5Cl] x H2O
   ▪ Standard Al-Komplexe: Locron P (Clariant), Micro-Dry 323 (Summit Reheis), Aloxicoll PF 40 (BK Giulini).
   ∘ Aktivierte Al-Komplexe: Reach 103 (Summit Reheis), AACH-7171/AACH-7172 (Summit Reheis), Aloxicoll P (BK Giulini), Aloxicoll SD100 (BK Giulini)
- Aluminiumsesquichlorhydrat [Al2(OH)4,5Cl1,5] x H2O
   Standard Al-Komplexe: Aloxicoll 31P (BK Giulini)
- Aluminiumdichlorhydrat [Al2(OH)4Cl2] x H2O

Die Aluminiumsalze können in gemahlener Form oder auch als Hohlkugeln oder als Mischung dieser vorliegen. Die Dichte dieser Partikel liegt vorteilhaft im Bereich von 0,7 bis 2,0 g/cm³.

Erfindungsgemäße Aerosolzubereitungen umfassen daher auch ein oder mehrere Silikon Gums und partikuläre Wirkstoffe, insbesondere Aluminiumchlorohydrate, mit einer Partikeldichte im Bereich von 0,7 bis 2,0 g/cm³, da auch sie die Sprühkegelbildung verbessern.

Als partikuläre Wirkstoffe sind insbesondere Antitranspirantien und deren Suspendierhilfen auszuwählen.

Vorteilhaft kann auch die Verwendung von AT-Salz-Suspensionen bzw. -Gelen sein, bei denen pulverförmig vorliegende Aluminiumsalze in diversen Ölen dispergiert angeboten werden.

Ebenso kann ein antimikrobieller Silbercitratkomplex, wie er in der DE 202008014407 beschrieben ist, bevorzugt als kosmetisch wirksamer Bestandteil in den kosmetischen Zubereitungen eingesetzt werden.

Die Antitranspirantwirkstoffe aus den zuvor geschilderten Gruppen werden in den erfindungsgemäßen Formulierungen bevorzugt in einer Menge von 0,5 bis 10 Gew.%, bevorzugt 2 bis 6 Gew.%, bezogen auf die Gesamtmasse der Zubereitung, d.h. inklusive der ggf. vorhandenen Treibgase, eingesetzt.
Bei Einsatz von ca. 35 Gew.% AACH (aktiviertes Aluminiumchlorohydrat) in der Wirkstofflösung für ein Aerosolspray (ohne Treibgas) und einem Abfüllverhältnis von etwa 15 : 85 (Wirkstofflösung zu Treibgas) wird ein Anteil von etwa 5,25 Gew. % AACH im Endprodukt vorhanden sein.
Bevorzugt werden in Antitranspirant-Aerosolzubereitungen Aluminiumchlorohydrate, insbesondere aktivierte Aluminiumchlorohydrate (AACH), als AT-Wirker eingesetzt.
In AT-Aerosolen sollten die Wirkstoffe leicht aufschüttelbar sein und während der Anwendung nicht zu schnell sedimentieren. Darüber hinaus sollten die Wirkstoffe, die AT-Partikel, am Wirkort (unter der Achsel) auftreffen und sich nicht in der Umgebung ungezielt verteilen. Diesem Umstand wird erfindungsgemäß durch eine erfindungsgemäße Zubereitung mit verbesserter Sprühkegelbildung genügt.
AT-Mittel aus der Gruppe der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis 1,0 Gew.%, vorzugsweise 0,1%-0,7%, insbesondere 0,3%-0,5 Gew.%, bezogen auf die Gesamtmasse der Zubereitung mit Treibgas zugesetzt werden. Selbstverständlich ist es möglich, weitere oder andere Antitranspirantwirkstoffe und/oder Deodorantien als kosmetisch wirksamen Bestandteil zu zusetzen.
Bevorzugt wird als kosmetisch wirksamer Bestanteil eine oder mehrere Verbindungen aus der Gruppe der Deodorantien, insbesondere Parfüme, Alkohole, antimikrobielle und/oder antitranspirant wirksame Wirkstoffe gewählt.
Bevorzugt umfassen die erfindungsgemäßen Aerosolzubereitungen ein oder mehrere Silikon Gums und ein oder mehrere Kohlenwasserstoffgemische mit einer überwiegenden Kettelänge größer/gleich C16 und b.) partikuläre Wirkstoffe, insbesondere Aluminiumchlorohydrat, mit einer Partikeldichte im Bereich von 0,7 bis 2,0 g/cm³.
Im erfindungsgemäßen Aerosol wird als Treibgas bevorzugt Propan, Propen, n-Butan, iso-Butan, iso-Buten, n-Pentan, n-Penten, iso-Pentan, iso-Penten, Methan und Ethan einzeln oder in Kombination eingesetzt. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt.
Besonders bevorzugt sind Propan, Butan, iso-Butan bzw. Mischungen dieser Treibgase. Die genannten Gase können im Sinne der vorliegenden Erfindung jeweils einzeln oder in beliebigen Mischungen zueinander verwendet werden. Bevorzugt werden Mischungen mit Druckstufen von 1,0 bis 3,5 bar eingesetzt. Bevorzugt 2,5 bis 3,0, ganz besonders bevorzugt ist eine Druckstufe von 2,7 bar.
Auch bei Verwendung von Treibmitteln hoher Druckstufe (> 2,1 bar) ist es mit den erfindungsgemäßen Zubereitungen möglich, den Feinanteil im Sprühstoß zu reduzieren. Bevorzugte Abfüllverhältnisse von Wirkstofflösung zu Treibgas betragen 40:60 bis 5 :95, bevorzugt 25:75 bis 10:90, ganz besonders bevorzugt 15:85.
Die Aerosolzubereitungen, insbesondere Deodorant- oder Antitranspirantzubereitungen, gemäß der Erfindung können kosmetische Hilfsstoffe und weitere Wirkstoffe enthalten, wie sie üblicherweise in Deodorant- oder Antitranspirantzubereitungen verwendet werden, wie z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften nicht beeinträchtigt oder erfindungsgemäß ausgeschlossen ist.
Bevorzugte Applikationsform für die erfindungsgemäßen Formulierungen sind wasserfreie Aerosole, die in Aluminiumdosen auf den Markt kommen. Bevorzugt ist diese Applikationsform, da sie weltweit die häufigste Form im Bereich Deo/AT darstellt. Auch aufgrund der sehr guten Packmittelkompatibilität ist ein Einsatz in wasserfreien Formulierungen zu empfehlen, da es auch nach Lagerung bei +40°C über einen Zeitraum von 6 Monaten kaum zu Packmittelveränderungen kommt.
Die nachfolgenden Beispiele illustrieren erfindungsgemäße Zubereitungen. Die Angaben sind Gewichtsanteile, bezogen auf die Gesamtmasse der jeweiligen Zubereitungen inkl. Treibgas. Die Beispiele 1-7 und 24-26 sind nicht erfindungsgemäß.

### Beispiele

| INCI | Standard AT-Spray | Bsp. 1 | Bsp. 2 | Bsp. 3 |
|---|---|---|---|---|
| Butyloctanoic Acid | 0.0375 | 0.0375 | 0.0375 | 0.0375 |
| Aluminum Chlorohydrate (Activated) | 5.25 | 5.25 | 5.25 | 5.25 |
| Cyclomethicone | 7.59 | 6.855 | 6.105 | 5.355 |
| Persea Gratissima Oil | 0.015 | | | |
| Cyclomethicone (85 %) + Dimethiconol (15 %) | --- | 0.75 (0.1125 % Aktivgehalt Silikon Gum) | 1.5 (0,225) | 2.25 (0,3375) |
| Octyldodecanol | 0.12 | 0.12 | 0.12 | 0.12 |
| Dimethicone | 0.45 | 0.45 | 0.45 | 0.45 |
| Parfum | 0.9375 | 0.9375 | 0.9375 | 0.9375 |
| Disteardimonium Hectorite | 0.6 | 0.6 | 0.6 | 0.6 |
| Butane + Isobutane + Propane | 85 | 85 | 85 | 85 |
| Summe | 100 | 100 | 100 | 100 |
| Anteil Partikel mit Durchmesser <10µm in % | 44 | 21 | 18 | 13 |

| INCI | Bsp. 4 | Bsp. 5 | Bsp. 6 | Bsp. 7 |
|---|---|---|---|---|
| Aluminum Chlorohydrate | | | 2,505 | |
| Butyloctanoic Acid | 0,0375 | 0,0375 | 0,0375 | 0,0375 |
| Aluminum Chlorohydrate (Activated) | 5,25 | 5,25 | | 5,25 |
| Cyclomethicone | 6,855 | 3,84 | 7,296 | 6,855 |
| Persea Gratissima Oil | | 0,015 | 0,0015 | |
| Isopropyl Palmitate | | 1,5 | 1,5 | |
| Cyclomethicone (85 %) + Dimethiconol (15 %) | 0,75 (0,1125) | 0,75 (0,1125) | 0,75 (0,1125) | 0,75 (0,1125) |
| C12-15 Alkyl Benzoate | | 1,5 | 1,5 | |
| Octyldodecanol | 0,12 | 0,12 | | 0,12 |
| Dimethicone | 0,45 | 0,45 | | 0,45 |
| Parfum | 0,9375 | 0,9375 | 0,99 | 0,9375 |
| Propylene Carbonate | | | 0,12 | |
| Disteardimonium Hectorite | 0,6 | 0,6 | 0,3 | 0,6 |
| Butane + Isobutane + Propane | 85 | 85 | 85 | 85 |
| Summe | 100 | 100 | 100 | 100 |
| Anteil Partikel mit Durchmesser <10µm in % | 25 | 22 | 21 | 26 |

Die Partikelgrößenverteilung wurde für alle Beispiele mit gleichem Ventil und Sprühkopf vermessen.

| | Bsp.8 | Bsp.9 | Bsp.10 |
|---|---|---|---|
| Aluminum Chlorohydrate (Activated) | 5,25 | 5,25 | 5,25 |
| Parfum | 0,94 | 0,94 | 0,94 |
| Cyclomethicone (85%) + Dimethiconol (15%) | 1,50 (0,225) | | |
| Dimethicone (88% M<100,000) + Dimethiconol (12%) | | 1,50 (0,18) | |
| C13-16 Isoparaffin + Dimethicone (20%) + C10-13 Isoparaffin | | | 1,50 (0,3) |
| Octyldodecanol | 0,15 | 0,15 | 0,15 |
| Dimethicone (M<100,000) | 3,51 | 3,51 | 3,51 |
| C 14-22 Alkane | 3,06 | 3,06 | 3,06 |
| Disteardimonium Hectorite | 0,60 | 0,60 | 0,60 |
| Butane + Isobutane + Propane | 85,00 | 85,00 | 85,00 |
| Summe | 100 | 100 | 100 |
| Anteil Partikel mit Durchmesser <10µm in % | 18 | 22 | 10 |

| | Bsp. 11 | Bsp. 12 | Bsp. 13 | Bsp. 14 | Bsp. 15 | Bsp. 16 |
|---|---|---|---|---|---|---|
| Aluminum Chlorohydrate (Activated) | 5,25 | 5,25 | 5,25 | 5,25 | 5,25 | 5,25 |
| Parfum | 0,94 | 0,94 | 0,94 | 0,94 | 0,94 | 0,94 |
| Disteardimonium Hectorite | 0,60 | 0,60 | 0,30 | 0,60 | 0,60 | 0,60 |
| Cyclomethicone (85%) + Dimethiconol (15%) | 1,50 (0,225) | | | | | |
| Dimethicone (88%, M<100,000 g/mol) + Dimethiconol (12%) | | 1,50 (0,18) | | 0,94 (0,1128) | | 0,94 (0,1128) |
| C13-16 Isoparaffin + Dimethicone (20%) + C10-13 Isoparaffin | | | 0,56 (0,112) | | 0,56 (0,112) | |
| Octyldodecanol | 0,15 | 0,15 | | | | |
| Dimethicone (M<100,000) | 0,45 | 0,45 | | | | 1,50 |
| Caprylic/Capric Triglyceride | 3,06 | 3,06 | 3,81 | 3,00 | 3,15 | 2,89 |
| C 14-22 Alkane | 3,06 | 3,06 | 4,14 | 4,28 | 4,50 | 2,89 |
| Butane + Isobutane + Propane | 85,00 | 85,00 | 85,00 | 85,00 | 85,00 | 85,00 |
| Summe | 100 | 100 | 100 | 100 | 100 | 100 |
| Anteil Partikel mit Durchmesser <10µm in % | 16 | 19 | | 24 | 22 | 19 |

| | Bsp. 17 | Bsp. 18 | Bsp. 19 | Bsp. 20 | Bsp. 21 | Bsp. 22 | Bsp. 23 |
|---|---|---|---|---|---|---|---|
| Aluminum Chlorohydrate (Activated) | 5,25 | 5,25 | 5,25 | 5,25 | 5,25 | 5,25 | 5,25 |
| Parfum | 0,94 | 0,94 | 0,94 | 0,94 | 0,94 | 0,94 | 0,94 |
| Disteardimonium Hectorite | 0,60 | 0,60 | 0,60 | 0,60 | 0,60 | 0,60 | 0,60 |
| Dimethicone (88%, M<100,000) + Dimethiconol (12%) | 1,50 (0,18) | 0,94 (0,1128) | 0,94 (0,1128) | 0,94 (0,1128) | 0,94 (0,1128) | 0,94 (0,1128) | 0,94 (0,1128) |
| Hydrogenated Polyisobutene | 3,06 | 2,89 | | | | 5,78 | 4,33 |
| Octyldodecanol | 0,15 | | | | | | |
| Dimethicone (M<100,000) | 0,45 | 1,50 | 1,50 | 1,50 | 1,50 | 1,50 | 1,50 |
| Caprylic/Capric Triglycerid | 3,06 | 2,89 | 2,89 | 1,44 | | | 1,44 |
| C 14-22 Alkane | | | 1,44 | 2,89 | 2,89 | | |
| Helianthus Annuus Seed Oil | | | 1,44 | 1,44 | 2,89 | | |
| Butane + Isobutane + Propane | 85,00 | 85,00 | 85,00 | 85,00 | 85,00 | 85,00 | 85,00 |
| Summe | 100 | 100 | 100 | 100 | 100 | 100 | 100 |
| Anteil Partikel mit Durchmesser <10µm in % | 12 | 16 | 19 | 20 | 18 | 21 | 18 |

| INCI | Bsp. 24 | Bsp. 25 | Bsp. 26 |
|---|---|---|---|
| Butyloctanoic Acid | 0.0375 | 0.0375 | 0.0375 |
| Aluminum Chlorohydrate (Activated) | 5.25 | 5.25 | 5.25 |
| Cyclomethicone | 4.59 | 6.855 | 6.105 |
| Persea Gratissima Oil | 0.015 | | |
| Isopropyl Palmitate | 1.5 | 1.5 | 1.5 |
| Cyclomethicone (85 %) + Dimethiconol (15 %) | 0.6 (0.09% Aktivgehalt Silikon Gum) | 0,45% (0.0675% Aktivgehalt Silikon Gum) | 0,3% (0.045%) |
| C12-15 Alkyl Benzoate | 1.5 | 1.5 | 1.5 |
| Octyldodecanol | 0.12 | 0.12 | 0.12 |
| Dimethicone | 0.45 | 0.45 | 0.45 |
| Parfum | 0.9375 | 0.9375 | 0.9375 |
| Disteardimonium Hectorite | 0.6 | 0.6 | 0.6 |
| Butane + Isobutane + Propane | 85 | 85 | 85 |
| Summe | 100 | 100 | 100 |
| Anteil Partikel mit Durchmesser <10µm in % | 22 | 23 | 27 |

| | Bsp. 27 Standard (Stand der Technik) | Bsp. 28 | Bsp. 29 | Bsp. 30 | Bsp. 31 |
|---|---|---|---|---|---|
| Kohlenwasserstoff Öl mit mindestens 100% ≥C16 | 4,155 | 4,061 | 3,968 | 3,874 | 3,780 |
| Dimethicone (86% M<100,000) + Dimethicone (14%) | ---- | 0,188 (0,02632) | 0,375 (0,0525) | 0,563 (0,07882) | 0,750 (0,105) |
| Aluminum Chlorohydrate | 5,250 | 5,250 | 5,250 | 5,250 | 5,250 |
| Caprylic/Capric Triglyceride | 2,078 | 2,031 | 1,984 | 1,937 | 1,890 |
| Dimethicone | 2,078 | 2,031 | 1,984 | 1,937 | 1,890 |
| Parfum | 0,990 | 0,990 | 0,990 | 0,990 | 0,990 |
| Propylene Carbonate | 0,075 | 0,075 | 0,075 | 0,075 | 0,075 |
| Disteardimonium Hectorite | 0,375 | 0,375 | 0,375 | 0,375 | 0,375 |
| Butane + Isobutane + Propane | 85 | 85 | 85 | 85 | 85 |
| Anteil Partikel mit Durchmesser <10µm in % | 44,4 | 31,7 | 26,1 | 22,1 | 19,5 |

In der nachfolgenden Liste sind die verwendeten Rohstoffe und deren Handelsbezeichnungen benannt:

| INCI | Handelsname | Hersteller/Lieferant |
|---|---|---|
| Aluminum Chlorohydrate | Aloxicoll PF 40 | BK Giulini Chemie |
| Butyloctanoic Acid | Isocarb 12 | Sasol |
| Aluminum Chlorohydrate (Activated) | Activated Aloxicoll P | BK Giulini Chemie |
| Cyclomethicone | Baysilone SF 1202 | Momentive Performance Materials Inc, |
| Persea Gratissima Oil | Avocado Oil, raff,DAC | Henry Lamotte |
| Isopropyl Palmitate | Isopropylpalmitate | Cognis |
| Cyclomethicone + Dimethiconol | DC 1501 Fluid | Dow Corning |
| Cyclomethicone + Dimethicone | DC 1411 Fluid | Dow Corning |
| Dimethicone + Dimethiconol | DC 1503 Fluid | Dow Corning |
| C13-16 Isoparaffin + Dimethicone + C10-13 Isoparaffin | DC BY 25-320 | Dow Corning |
| Dimethicone + Dimethicone | DC 1413 Fluid | Dow Corning |
| C12-15 Alkyl Benzoate | Tegosoft TN 2 | Evonik Goldschmidt |
| Octyldodecanol | Isofol 20 | Sasol |
| Dimethicone (M <100,000 g/mol) | BRB Silikone Oil DM 100 | BRB International |
| Propylene Carbonate | 807051 Propylencarbonat zur Synthese | Merck |
| Disteardimonium Hectorite | Bentone 38V CG | Elementis |
| Butane + Isobutane + Propane | Drivosol 27 D60 | Evonik Oxeno |

## Patentansprüche

1. Kosmetische oder dermatologische wasserfreie Antitranspirant-Aerosolzubereitung umfassend ein oder mehrere Silikon Gums und
- ein oder mehrere Kohlenwasserstoff-Gemische mit einem Anteil an Kohlenwasserstoffen mit einer Kettenlänge größer und/oder gleich C16 von mindestens 50 Gew.%, bezogen auf die Gesamtmasse des Kohlenwasserstoffgemisches,
**dadurch gekennzeichnet, dass** der Anteil an Cyclomethiconen, weniger als 8 Gew.%, bezogen auf die Gesamtmasse der Zubereitung, beträgt.

2. Zubereitung nach Anspruch 1 **dadurch gekennzeichnet, dass** der Anteil an Silikon Gum im Bereich von 0,02 - 0,5 Gew.%, bevorzugt im Bereich von 0,035 % bis 0,2 Gew.%, insbesondere im Bereich von 0,04 bis 0,1 Gew.%, bezogen auf die Gesamtmasse der Zubereitung, gewählt wird.

3. Zubereitung nach einem der vorstehenden Ansprüche **dadurch gekennzeichnet, dass** sie zusätzlich enthalten ein oder mehrere Lipide gewählt aus der Gruppe der Triglyceride, bevorzugt Caprylic/Capric Triglyceride.

4. Zubereitung nach einem der vorstehenden Ansprüche **dadurch gekennzeichnet, dass** die Aerosolzubereitung partikuläre Wirkstoffe mit einer Partikeldichte im Bereich von 0,7 bis 2,0 g/cm³ umfasst.

5. Zubereitung nach Anspruch 4 **dadurch gekennzeichnet, dass** als partikuläre Wirkstoffe Aluminiumchlorohydrate gewählt werden.

6. Zubereitung nach Anspruch 5 **dadurch gekennzeichnet, dass** als Aluminiumchlorohydrate aktivierte Aluminiumchlorohydrate (AACH) gewählt werden.

7. Zubereitung nach einem der vorstehenden Ansprüche **dadurch gekennzeichnet, dass** Suspendierhilfsmittel in der Zubereitung enthalten sind.

8. Zubereitung nach einem der vorstehenden Ansprüche **dadurch gekennzeichnet, dass** der Anteil an flüchtigen Silikonölen weniger als 8 Gew.%, insbesondere 0 Gew.%, bezogen auf die Gesamtmasse der Zubereitung, beträgt.

9. Zubereitung nach einem der vorstehenden Ansprüche **dadurch gekennzeichnet, dass** der Anteil der Kohlenwasserstoff-Gemische in der Aerosolzubereitung 0,3 bis 15,0 Gew.%, insbesondere 0,4 bis 10,0 Gew.%, bezogen auf die Gesamtmasse der Zubereitung, beträgt.

10. Zubereitung nach einem der vorstehenden Ansprüche **dadurch gekennzeichnet, dass** das Abfüllverhältniss von Wirkstofflösung zu Treibgas 40:60 bis 5 :95, bevorzugt 25:75 bis 10:90, ganz besonders bevorzugt 15:85, beträgt.

## Claims

1. Cosmetic or dermatological anhydrous antiperspirant aerosol preparation comprising one or more silicone gums and
- one or more hydrocarbon mixtures with a proportion of hydrocarbons having a chain length greater than and/or equal to C16 of at least 50% by weight, based on the total mass of the hydrocarbon mixture,
**characterized in that** the proportion of cyclomethicones is less than 8% by weight, based on the total mass of the preparation.

2. Preparation according to Claim 1, **characterized in that** the proportion of silicone gum is selected in the range of 0.02 - 0.5% by weight, preferably in the range of 0.035% to 0.2% by weight, especially in the range of 0.04 to 0.1% by weight, based on the total mass of the preparation.

3. Preparation according to either of the preceding claims, **characterized in that** said preparation additionally comprises one or more lipids selected from the group of triglycerides, preferably caprylic/capric triglycerides.

4. Preparation according to any of the preceding claims, **characterized in that** the aerosol preparation comprises particulate active ingredients having a particle density in the range of 0.7 to 2.0 g/cm³.

5. Preparation according to Claim 4, **characterized in that** aluminium chlorohydrates are selected as particulate active ingredients.

6. Preparation according to Claim 5, **characterized in that** the aluminium chlorohydrates selected are activated aluminium chlorohydrates (AACH).

7. Preparation according to any of the preceding claims, **characterized in that** suspension aids are present in the preparation.

8. Preparation according to any of the preceding claims, **characterized in that** the proportion of volatile silicone oils is less than 8% by weight, especially 0% by weight, based on the total mass of the preparation.

9. Preparation according to any of the preceding claims, **characterized in that** the proportion of the hydrocarbon mixture in the aerosol preparation is 0.3 to 15.0% by weight, especially 0.4 to 10.0% by weight, based on the total mass of the preparation.

10. Preparation according to any of the preceding claims, **characterized in that** the fill ratio of active ingredient solution to propellant is from 40:60 to 5:95, preferably from 25:75 to 10:90, especially preferably 15:85.

## Revendications

1. Préparation cosmétique ou dermatologique antitranspirante anhydre en aérosol, comprenant une ou plusieurs gommes de silicone et
- un ou plusieurs mélanges d'hydrocarbures contenant une proportion d'hydrocarbures ayant une longueur de chaîne supérieure et/ou égale à C16 d'au moins 50 % en poids, par rapport à la masse totale du mélange d'hydrocarbures,
**caractérisée en ce que** la proportion de cyclométhicones est de moins de 8 % en poids, par rapport à la masse totale de la préparation.

2. Préparation selon la revendication 1, **caractérisée en ce que** la proportion de gommes de silicone est choisie dans la plage allant de 0,02 à 0,5 % en poids, de préférence dans la plage allant de 0,035 à 0,2 % en poids, notamment dans la plage allant de 0,04 à 0,1 % en poids, par rapport à la masse totale de la préparation.

3. Préparation selon l'une quelconque des revendications précédentes, **caractérisée en ce qu'**elle contient en outre un ou plusieurs lipides choisis dans le groupe des triglycérides, de préférence des triglycérides capryliques/capriques.

4. Préparation selon l'une quelconque des revendications précédentes, **caractérisée en ce que** la préparation en aérosol comprend des agents actifs particulaires ayant une densité de particule dans la plage allant de 0,7 à 2,0 g/cm³.

5. Préparation selon la revendication 4, **caractérisée en ce que** des chlorohydrates d'aluminium sont choisis en tant qu'agents actifs particulaires.

6. Préparation selon la revendication 5, **caractérisée en ce que** des chlorohydrates d'aluminium activés (AACH) sont choisis en tant que chlorohydrates d'aluminium.

7. Préparation selon l'une quelconque des revendications précédentes, **caractérisée en ce que** des adjuvants de suspension sont contenus dans la préparation.

8. Préparation selon l'une quelconque des revendications précédentes, **caractérisée en ce que** la proportion d'huiles de silicone volatiles est inférieure à 8 % en poids, notamment de 0 % en poids, par rapport à la masse totale de la préparation.

9. Préparation selon l'une quelconque des revendications précédentes, **caractérisée en ce que** la proportion des mélanges d'hydrocarbures dans la préparation en aérosol est de 0,3 à 15,0 % en poids, notamment de 0,4 à 10,0 % en poids, par rapport à la masse totale de la préparation.

10. Préparation selon l'une quelconque des revendications précédentes, **caractérisée en ce que** le rapport de remplissage entre la solution d'agents actifs et le gaz propulseur est de 40:60 à 5:95, de préférence de 25:75 à 10:90, de manière tout particulièrement préférée de 15:85.
